Europäisches Patentamt

European Patent Office

Office européen des brevets

(11) Publication number: **0 151 018**
**B1**

## EUROPEAN PATENT SPECIFICATION

(45) Date of publication of patent specification: **22.03.89**

(21) Application number: **85300569.2**

(22) Date of filing: **28.01.85**

(51) Int. Cl.⁴: **A 61 F 13/18**, B 32 B 9/02

54 **Absorbent laminates.**

(30) Priority: **26.01.84 GB 8402095**

(43) Date of publication of application:
**07.08.85 Bulletin 85/32**

(45) Publication of the grant of the patent:
**22.03.89 Bulletin 89/12**

(84) Designated Contracting States:
**AT BE CH DE FR IT LI LU NL SE**

(56) References cited:
**DE-A-2 503 469**
**DE-A-3 214 354**
**GB-A-2 081 320**
**NZ-A- 183 103**
**US-A-3 371 667**
**US-A-3 545 442**
**US-A-3 811 445**

(73) Proprietor: **JOHNSON & JOHNSON**
**One Johnson & Johnson Plaza**
**New Brunswick, NJ 08933-7003 (US)**

(72) Inventor: **Brassington, Nigel John**
**13 Airedale View Cross Hills**
**Keighley North Yorkshire (GB)**

(74) Representative: **Jones, Alan John et al**
**CARPMAELS & RANSFORD 43 Bloomsbury**
**Square**
**London, WC1A 2RA (GB)**

**Description**

This invention relates to absorbent laminates, especially for use as or in products for absorbing body fluids, such as diapers, incontinence pads, sanitary napkins, tampons and wound dressings.

Characteristics which are desirable in most absorbent products are high absorbency (i.e. the capacity to absorb large quantities of fluid per unit weight of absorbent), and a high rate of fluid uptake. It is generally also desirable for the absorbent material to have a high degree of fluid retention, so that absorbed fluid is not readily released when the product is subjected to externally applied pressure.

New Zealand Patent Application No. 183103 discloses an absorbent laminate formed by needling a hydrophobic textile layer to a cellulosic absorbent layer.

US—A—3 545 442 discloses an absorbent bandaging and dressing material comprising at least two, and preferably three, or possibly more, layers of different types of materials. All of these layers are preferably formed of fibres which may be interlocked into a continuous fabric, e.g. by needling. A preferred structure comprises a first layer of polypropylene fibres, a second layer of cellulosic fibres and a third layer of rayon fibres. It is said that the second layer of such a structure may be used primarily for the purpose of holding the highly absorbent fibres of the third layer in place.

According to the present invention there is provided an absorbent laminate comprising a core layer sandwiched between two facing layers, said core layer comprising an absorbent peat moss board, said two facing layers each comprising a fluid-permeable web of hydrophobic fibres, said core layer and said two facing layers being consolidated into said laminate structure by a plurality of fibres extending from said facing layers into said core layer.

The absorbent laminates of the present invention not only have high absorbency and a high degree of fluid retention, but also the capacity to transfer fluid rapidly away from a facing layer which is initially wetted, into the core layer, leaving the facing layer substantially dry to the touch. This latter characteristic is especially desirable in incontinence aids, because it gives greatly increased user comfort.

Fibres which extend from the two facing layers into the core layer serve as wicks along which aqueous fluids may be carried. Importantly, however, they also serve to increase the strength of the peat moss board, which is otherwise rather friable. The operation of introducing wicks into the peat moss board has the additional effect of rendering it more flexible.

As will be appreciated by those skilled in the art, the term "hydrophilic" is used to describe materials which are wetted by water, (i.e. materials which have contact angles with water less than 90°), while the term "hydrophobic" is used to describe materials which are not wetted by water (i.e. materials which have contact angles equal to or greater than 90°).

Examples of fibrous materials which may be used for the facing layers and for the wicks extending into the core layer are polyolefins such as polyethylene, polypropylene and polybutylene homopolymers and copolymers, vinyl polymers such as polyvinylchloride, polyamides such as nylon, and polyesters. Of course, it is also possible to use fibres of materials which are in themselves hydrophilic, if such fibres are first surface-treated in order to render them hydrophobic.

The two facing layers may be of the same materials as each other, or of different materials, and of the same or different thickness and/or weight. For example, the core layer may have one face covered with a polyester fibre web, and the other face covered with a viscose fibre web.

Preferably, each facing layer is in the form of a non-woven fibrous fleece, which may, if desired, be pre-needled in order to increase its coherence.

A material which is particularly preferred for the core layer is a peat moss composite board as described in British Patent Specification No. 2081320.

The wicks which extend from the facing layer into the core layer may be formed in a number of ways. Such wicks may be formed, for example, by stitch bonding the layers together. Preferably, however, the hydrophobic fibres of the facing layer are themselves used to form the wicks by needling the facing layer to the core layer. Needling generally involves passing barbed needles into and out of a fibrous web in a reciprocating motion. Fibres from within the web are caught on the barbs of the needles, and are either pushed forward with the forward movement of the needle, or drawn back with the reverse movement of the needle, or both, depending on the type of needle used. The density of needling is chosen according to the strength and fluid transport properties which are desired in the resulting laminate. Increasing the density of needling increases the number of wicks extending between the layers, and thereby increases the rate of fluid transfer from the facing layers to the core layer. Increasing the density of needling also increases resistance to delamination.

On the other hand, increased density of needling also tends to produce greater compaction of the absorbent layers, resulting in a decrease of the overall absorbent capacity, as well as an increase in the rigidity of the resulting product. This latter factor is of particular importance in cases where the increased rigidity may lead to user discomfort, such as may be the case, for example, if the laminates of the invention are used as incontinence pads.

Preferably, needling will be conducted at a density of from 5 to 20 needles/cm$^2$, while a needle density of from 5 to 15 needles/cm$^2$ is particularly preferred.

For some purposes, it will be appropriate to employ a needling density which is not constant over the whole area of the laminate. For example, a higher density of needling may be employed at the edges of an absorbent pad according to the invention, since it is here that greatest resistance to delamination is most likely to be required. Thus, it may be appropriate to needle a pad for use as a wound dressing at a density of 8 needles/cm² over its central area, and at a density of 12 or more needles/cm² at its edges.

The laminate may be needled on one or both faces of the core layer, and the needles may penetrate the core layer completely or just partially.

Because peat moss board is relatively friable, we have found that standard needling processes can lead to laminates with a machine direction strength which may be inadequate for certain purposes, such as when the laminates are used as incontinence pads. When such pads are worn by ambulant patients, considerable shear forces can be set up in the machine direction by the action of walking, resulting in the pad being stretched. When an unstrengthened needled laminate is used, this stretching can cause the absorbent layer to fracture. Such fracture is highly deleterious to the absorbent properties of the laminate, such as its strike-through time and the speed of surface drying. This type of fracture can be made less likely by strengthening the laminate. Strengthening can be provided by including within the laminate an extra layer comprising an inextensible or relatively inextensible web or net. However, a greatly preferred method is to include strengthening threads along the length (i.e. along the machine direction) of the laminate. These threads should be "furry", for example threads of the type called "Boucle". The furry exterior of these threads can then become entangled within the needled wicks passing perpendicularly through the laminate. The result of providing such threads at 1 cm intervals is to more than double the force required to fracture peat moss board, thus rendering the laminate more suitable for use as incontinence pads.

It will be understood that needling, although preferred, is not the only means by which fibres from within the facing layers can be caused to form wicks in accordance with the invention. For example, wicks may be formed by means of very fine, high pressure water jets directed at one or both free faces of two superimposed fibrous webs, as more fully described in United States Patent Specifications Nos. 2 862 251 and 3 033 721.

The absorbent capacity required of the laminate will, of course, depend on the end use to which it is to be put. Generally, however, it is preferred that both the core layer and the facing layers are capable of absorbing more than 5 grams of water per gram of absorbent. More preferably, the absorbent capacities of the core layer and the facing layers are both greater than 8 grams of water/grams of absorbent, for example 10 or more grams of water/grams of absorbent.

The facing layers should preferably be extremely permeable to fluids, so as to allow passage of fluid under gravity at a rate of at least 10 ml of fluid/second/cm² without surface puddling, and more preferably at greater than 20 ml/sec/cm², e.g. 30 ml/sec/cm². This is especially desirable when the laminate is used as an incontinence pad, so that urine can be absorbed even at full flow.

It will be appreciated that the absorbent products according to the present invention may incorporate one or more further layers in addition to the three layers described above.

A particularly advantageous product according to the invention comprises a central core of peat moss board, such as BISHOP (Trade Mark) board, sandwiched between webs of polyester fibre. A needled laminate of peat moss board with non-woven polyester fibre webs is found to have high tensile strengths, even when wet, in both the machine and cross directions, especially when reinforced by Bouclé threads as above described. Moreover, the laminate is soft and conformable. The polyester fibre webs are capable of absorbing fluids at extremely high rates, and such absorbed fluid is quickly transferred to the peat moss board by means of the wicks formed during the needling operation. Thus, the polyester fibre webs are found to dry out within a very short time (e.g. of the order of 1 minute) of being wetted. The product is also found to have a highly delayed strike-through rate. It is believed that this is part due to the swelling of the peat moss board on wetting, such swelling serving to restrict the pores formed through the board by the needling operation, thereby preventing wicking through to the opposite face of the product from the face which is wetted.

Peat moss board has the additional advantage of being somewhat deodorant, which is of great value in certain absorbent products, particularly incontinence pads and sanitary napkins.

Although peat moss board is naturally friable and produces a substantial quantity of fines, the open fluffy nature of the outer polyester webs tends to trap any fines generated, without the need for any coverstock. This is especially so if the peat moss board is entirely enclosed by the polyester fibre web, as is preferred for incontinence pads. The benefit of fully enclosing the peat moss board is largely aesthetic, but a secondary advantage is that damp or wet absorbent material is prevented from contacting the wearer's skin or clothing. Such a fully enclosed structure may be formed by intermittently feeding sections of peat moss board onto a continuous web of polyester fibre, covering the peat moss board with a second continuous polyester fibre web, needling and then severing the resulting laminate between the sections of peat moss board.

The absence of any coverstock can be advantageous in certain applications, such as when the absorbent product is used as an incontinence pad. Coverstocks reduce the rate of fluid uptake, add considerably to the cost and can lead to abrasion. Where a high rate of fluid uptake is less critical, such as in sanitary napkins, a coverstock can be used to enhance the appearance of the product.

When a laminate comprising peat moss board and a loose polyester fibre web are used in a wound dressing, it is preferable to provide a low adherence wound contacting surface, such as a spun bound polyester fabric.

In general, peat moss board will be used in weights of from 100g/m² to 1kg/m², and preferably from 300 to 500g/m². As mentioned above, however, the actual weight used, and that of the facing layers, will depend on the end use to which the laminate is to be put. For sanitary protection aids or wound dressings, for example, it is preferred to use a light weight peat moss board (160g/m²) surrounded by the lightest weight polyester fleece that is practical (e.g. 80g/m²). The density of needling should also be fairly light (e.g. 8 needles/cm²) to give a noticeably "furry" surface, the preferred polyester fleeces also being pre-needled at this density. This construction is preferred for sanitary protection aids and wound dressings for the following reasons: The fleece has a surface which can contact well with any coverstock placed over it; the very light fleece is adequate to obscure the peat moss board and to trap any fines; the laminate is not too bulky, and is highly flexible and conformable; the thin polyester fleece has very rapid surface drying properties (the surface being dry to the touch within a few seconds of being wetted).

For incontinence pads it is preferred to use a heavier weight peat moss board surrounded by a thicker polyester fleece which has been more heavily pre-needled. The preferred construction is a 360g/m² microcreped peat moss board with 180g/m² polyester fleeces (pre-needled at 12 needles/cm²) laminated on both sides using 12 needles/cm². This construction has the following advantages: The total absorbency is appropriate to the needs of patients with stress or other forms of light voiding incontinence; the surface is coherent and does not require a coverstock; the fleeces are thick enough to hold considerable quantities of fluid in the short time before it is fully absorbed by the peat moss board; the thicker fleeces also give a high degree of cushioning.

For incontinence pads for patients with detrusor instability or otherforms of rapid voiding incontinence, it is preferred to use even heavier peat moss board (e.g. 500 g/m²) or multiple layers of lighter board (e.g. two or three layers of 330 g/m² board).

Absorbent laminates according to the present invention are now described, by way of example, with reference to the accompanying drawings, in which:

Figure 1 is a plan view of an absorbent laminate according to the invention;

Figure 2 is a schematic section on line A—A of Figure 1, and

Figure 3 is a schematic section through an alternative absorbent laminate.

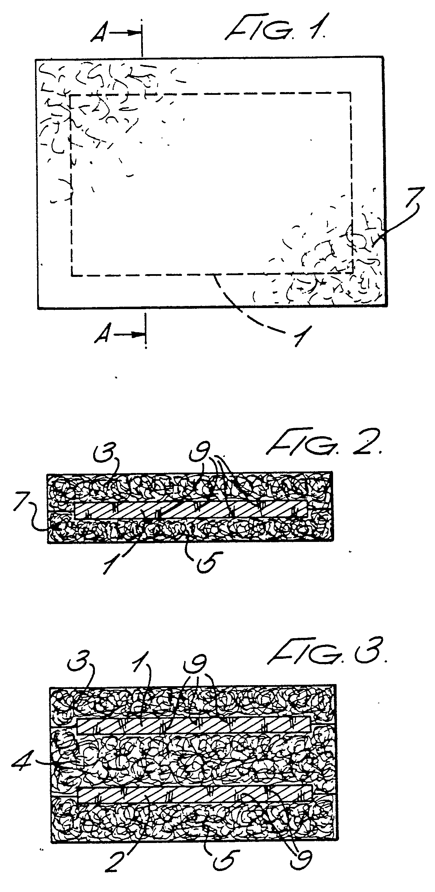

Referring to Figures 1 and 2 of the drawings, an absorbent laminate comprises a core layer 1, sandwiched between two facing layers 3, 5. The core layer 1 is a rectangular section of peat moss board of weight 330g/m², manufactured according to British Patent Specification No. 2 081 320. This material is typically able to absorb 10 times its own weight of water. The facing layers 3, 5 are both non-woven pre-needled polyester fibre fleeces of weight 250g/m², (Cosmopolitan Textiles Ltd., Winsford, Cheshire, England). Fleeces of widely different weights could, of course, be used, but weights within the range 100 to 500 g/m² are preferred. Each of the facing layers overlaps all four sides of the core layer, so that the latter is entirely enclosed and is surrounded by an edge region 7.

The three layers 1, 3, 5 are joined by needling at a density of 8 needles/cm². This needling operation forms wicks 9 of polyester fibre which extend from the facing layers 3, 5 into the core layer 1. The edge region 7 is needled at a density of 12 needles/cm², to form a strong bond between the peripheral areas of the facing layers.

Figure 3 illustrates a laminate which is analogous to that of Fibres 1 and 2, but differs in that two core layers of peat moss board 1, 2 are provided, with three facing layers 3, 4, 5 of polyester fibre web.

The present invention is further illustrated by the following examples.

## Example 1

A three layer laminate is formed from the following materials:

Layer 1.  Pre-needled (8 needles/cm²) polyester fleece, 130 g/m².
Layer 2.  Peat moss board, made as described in British Patent Specification No. 2 081 320, 330g/m².
Layer 3.  As layer 1.

The layers were joined by needling at 8 needles/cm², and the absorbent characteristics of the resulting laminate were tested by applying 20 mls of physiological saline solution to one surface of the laminate in one second. (This fluid is a synthetic aqueous composition containing 0.9% sodium chloride in distilled water). Within 2 minutes, the wetted surface of the laminate was found to be dry to the touch.

By way of comparison, a similar laminate which had not been needled was subjected to the same test. The wetted surface was found to remain damp to the touch for at least 10 minutes, and in some cases up to 2 hours after being wetted.

## Example 2

A laminate was prepared as described in Example 1, except that polyester fleeces of 100g/cm² were used in place of the heavier fleeces described therein. The strike-through time and absorbency of the laminate were tested as follows:

The laminate was sandwiched between two transparent PERSPEX (Trade Mark) plates, the lower of which was provided with a hole for the application of test fluid to the laminate under a small (e.g. 0.5 cm) hydrostatic head. The strike-through time of the laminate was the time taken for such applied fluid to contact the upper PERSPEX plate, as detected by the specular appearance of the latter when first wetted.

It was found that the strike-through time of the laminate was 180 seconds, at which time 18.0g of Meds fluid had been absorbed.

When a three-layer laminate was formed using 100g/m² viscose fleeces in place of polyester fleeces, the strike-through time was reduced to 15 seconds, at which time only 1.8g of Meds fluid had been absorbed. Moreover, the surface of this laminate remained damp to the touch, even several hours after having been wetted.

Example 3

This example shows the effects of different needle densities on absorbency and other properties of the laminate.

Two laminates were made from peat moss board (320g/m²) having polyester fleeces (100g/m²) needled to each surface. The first laminate was needled at 8 needles/cm² (light tack), and the second at 12 needles/cm² (medium tack), and the two products tested for absorptive capacity (Meds fluid), wicking ability and tensile strength. The results were as follows:

|  | LIGHT TACK | MEDIUM TACK |
| --- | --- | --- |
| Absorptive Capacity | 14.3g/g | 13.1g/g |
| Machine Direction Wicking | 6.2g | 6.4g |
|  | 9mm | 13mm |
| Cross Direction Wicking | 6.8g | 5.9g |
|  | 8mm | 12mm |
| Tensile strength (Machine Direction) | 1.1Kg/5cm | 1.0Kg/5cm |
| (Cross Direction) | 1.7Kg/5cm | 1.4Kg/5cm |

It will be seen that increased needle density increases the wicking distance of the laminate, but decreases the absorptive capacity and tensile strength.

The polyester fibre used in the above Examples was a surface-treated polyethylene terephthalate (ICI type 578). The surface-treatment was with a mixture of oleyl cetyl alcohol and castor oil ethylene oxide condensates with sodium benzoate, potassium hexyl phosphate and propanol.

It will be understood that the present invention is described above merely by way of example, and numerous variations may be made without departing from the scope of the invention.

**Claims**

1. An absorbent laminate comprising a core layer (1) sandwiched between two facing layers (3, 5), said core layer comprising an absorbent peat moss board, said two facing layers each comprising a fluid-permeable web of hydrophobic fibres, said core layer and said two facing layers being consolidated into said laminate structure by a plurality of fibres extending from said facing layers into said core layer.

2. An absorbent laminate according to claim 1 wherein the facing layers are formed from a polyolefin, a vinyl polymer, a polyamide or a polyester.

3. An absorbent laminate according to claim 1 or claim 2, further comprising reinforcing threads extending parallel to the major surfaces thereof.

4. A diaper, incontinence pad, sanitary napkin, tampon or wound dressing comprising or consisting of a laminate according to any preceding claim.

5. A method of making an absorbent laminate according to any of claims 1 to 3, wherein the core layer and two facing layers are consolidated by needling.

6. A method according to claim 5 wherein the laminate is consolidated by needling at a density of from 5 to 20 needles/cm².

**Patentansprüche**

1. Absorbieredes Laminat, umfassend eine Kernschicht (1), die zwischen zwei Abdecklagen (3, 5) eingelegt ist, wobei die genannte Kernschicht ein Blatt aus absorbierendem Torfmoos umfaßt, und wobei die genannten zwei Abdecklagen jeweils eine flüssigkeitsdurchlässige Bahn aus hydrophoben Fasern umfassen, und wobei die genannte Kernschicht und die genannten zwei Abdecklagen dadurch zu der genannten Laminatstruktur vereinigt sind, daß sich eine Mehrzal von Fasern von den genannten Abdecklagen in die genannte Kernschicht hinein erstreckt.

2. Absorbierendes Laminat nach Anspruch 1, worin die Abdecklagen aus einem Polyolefin, einem Vinylpolymer, einem Polyamid oder einem Polyester augebildet sind.

3. Absorbierendes Laminat nach Anspruch 1 oder Anspruch 2, welches weiterhin Verstärkungsfäden enthält, die sich parallel zu den Hauptoberflächen des Laminates erstrecken.

4. Ein Windel, Inkontinenzeinlage, Damenbinde, ein Tampon oder ein Wundverband, welche(r) ein Laminat nach einm der vorhergehenden Ansprüche enthält oder aus einem solchen besteht.

5. Verfahren zur Herstellung eines absorbierenden Laminates nach einem der Ansprüche 1 bis 3, bei welchem die Kernschicht und die zwei Abdecklagen durch Nadeln miteinander vereinigt werden.

6. Verfahren nach Anspruch 5, wobei das Laminat durch Nadeln in einer Dichte von 5 bis 20 Nadeln/cm² vereinigt wird.

**Revendications**

1. Une feuille absorbante laminée comprenant une couche centrale (1) intercalée entre deux couches externes (3, 5), ladite couche centrale comprenant une plaque absorbante en sphaigne, lesdites deux couches externes comprenant chacune un tissu perméable aux liquides en fibres hydrophobes, ladite couche centrale et lesdites deux couches externes étant consolidées dans ladite structure laminée par de nombreuses fibres qui s'étendent desdites couches externes jusque dans ladite couche centrale.

2. Une feuille absorbante laminée selon la revendication 1, dans laquelle les couches externes sont formées à partir d'une polyoléfine, d'un polymère de vinyle, d'un polyamide ou d'un polyester.

3. Une feuille absorbante laminée selon la revendication 1 ou 2, comprenant en plus des fils renforcés s'étendant parallèlement à leurs surfaces principales.

4. Une couche, une protection pour incontinents, une serviette hygiénique, un tampon ou un pansement comprenant ou composé d'une feuille laminée selon l'une des revendications précédantes.

5. Une méthode pour fabriquer une feuille absorbante laminée selon l'une des revendications 1 à 3, dans laquelle la couche centrale et les deux couches externes sont consolidées par une couture.

6. Une méthode selon la revendication 5 dans laquelle la feuille laminée est renforcée par couture à une densité de 5 à 20 points/cm².

EP 0 151 018 B1

FIG. 1.

FIG. 2.

FIG. 3.